Europäisches Patentamt

(19) European Patent Office

Office européen des brevets

(11) EP 0 855 377 A1

## (12) EUROPEAN PATENT APPLICATION

(43) Date of publication:
29.07.1998 Bulletin 1998/31

(51) Int. Cl.⁶: C07C 17/38

(21) Application number: 97300468.2

(22) Date of filing: 24.01.1997

(84) Designated Contracting States:
BE DE ES FR GB IT NL

(71) Applicant: UOP
Des Plaines, Illinois 60017-5017 (US)

(72) Inventors:
• Jan, Chwu-Ching
Elk Grove Village, Illinois 60007 (US)
• Kalnes, Tom N.
LaGrange, Illinois 60525 (US)

• Hibel, George R.
Schaumburg, Illinois 60193 (US)

(74) Representative:
Brock, Peter William et al
Urquhart-Dykes & Lord
1 Richfield Place
Richfield Avenue
Reading RG1 8EQ Berkshire (GB)

## (54) Process for the selective removal of organic nitrates from a halogenated organic stream

(57) A feed stream containing halogenated organic compounds and trace quantities of organic nitrates is treated to produce a stream comprising halogenated organic compounds substantially free from organic nitrates by the utilization of a hydrogenation zone operated at selective hydrogenation conditions in order to convert the organic nitrates to one or more water-soluble nitrogen compounds while minimizing the production of hydrogen halide compounds. The resulting water-soluble nitrogen compounds are thereafter removed by extraction with an aqueous stream to produce the desired product stream.

EP 0 855 377 A1

## Description

FIELD

The field of art to which this invention pertains is the selective removal of organic nitrates from a stream comprising halogenated organic compounds and organic nitrates to produce a stream comprising halogenated organic compounds essentially free from organic nitrates. More specifically, the invention relates to a process for treating a stream containing halogenated organic compounds and having trace quantities of organic nitrates to produce a stream comprising halogenated organic compounds free from organic nitrates by the utilization of a hydrogenation zone operated at selective hydrogenation conditions in order to convert the organic nitrates to water-soluble nitrogen compounds while minimizing the hydrodehalogenation of the halogenated organic compounds.

BACKGROUND

In US-A-5013424, a process is disclosed wherein a feedstock comprising halogenated organic compounds is contacted with hydrogen in a hydrogenation reaction zone to produce hydrocarbonaceous compounds and at least one water-soluble inorganic halide compound. The '424 patent contemplates processing a feedstock which is free of contaminating organic nitrates which would complicate the operation of the process to produce at least one water-soluble inorganic halide compound. The main thrust of the '424 patent is to essentially convert all of the halide compounds into water-soluble inorganic halide compounds. In the event that the feed to the '424 patent contains significant quantities of organic nitrates, the resulting hydrogen halide compound which is produced would be contaminated by water-soluble nitrogen compounds and its value would thereby be decreased. In addition, the water-soluble nitrogen compounds would react with the co-produced hydrogen halide to form ammonium chloride which forms deposits in the processing plant when the hydrogenation zone effluent is cooled.

Recent developments in the treatment of streams containing halogenated organic compounds has created a demand for technology which is capable of treating a stream containing halogenated organic compounds and organic nitrates to selectively convert the organic nitrates to water-soluble nitrogen compounds while minimizing the production of hydrogen halide compounds. With the increased environmental emphasis for the treatment and recycle of waste streams containing organic compounds, there is an increased need for improved processes to accomplish such treatment and recycle. For example, during the disposal or recycle of potentially harmful hydrocarbonaceous waste streams, an important step in the total solution to the problem is the pretreatment or conditioning of an organic stream which facilitates the ultimate resolution to produce product streams which may subsequently be handled in an environmentally acceptable manner. Therefore, those skilled in the art have sought to find feasible techniques to remove organic nitrate compounds from a stream containing halogenated organic compounds and organic nitrate compounds to produce a stream comprising halogenated organic compounds essentially free from organic nitrates which may then be further treated or processed if desired.

It has recently been discovered that when a feedstock comprising halogenated organic compounds and relatively small quantities of organic nitrate compounds is processed to produce water-soluble inorganic halide compounds, several problems are encountered as a result of the conversion of organic nitrate compounds to ammonia and subsequently ammonium chloride. These problems include the need for higher operating temperatures to maintain inorganic halide production, the undesirable contamination of the inorganic halide compound product stream with nitrogen compounds and the plating out of ammonium chloride on the cooler surfaces of the plant as the reactor effluent is cooled in preparation for subsequent separation and product recovery. In many cases, the recovered inorganic halide compound product stream is recycled to production facilities, such as chlorine production, for example, which require high-purity halide compounds without nitrogen contaminants.

SUMMARY

The present invention provides a treatment process to produce a product stream comprising halogenated organic compounds and essentially free from organic nitrates from a feed stream comprising halogenated organic compounds and organic nitrates by contacting the feed stream and hydrogen with a selective hydrogenation catalyst in a hydrogenation zone at hydrogenation conditions to convert the organic nitrates into water-soluble nitrogen compounds while effectively minimizing the production of hydrogen halide compounds. Important elements of the process are the ability to produce a stream containing halogenated organic compounds essentially free of organic nitrates thereby permitting the subsequent conversion of the nitrogen-free stream in a facile manner if desired and conversion of the organic nitrates into water-soluble nitrogen compounds which can be readily separated and recovered for further use or disposal as desired. In accordance with the present invention, "essentially free of organic nitrates" means preferably containing, on an elemental basis, less than 20 ppm nitrogen and more preferably less than 10 ppm nitrogen. The present invention enjoys the advantage of selectively purifying a stream containing halogenated organic compounds that is contaminated with organic nitrates in a convenient and economical manner. The results of this advantage include the maximum recovery of hydrogen halide in subsequent recovery procedures, reduced operating costs, production of higher quality hydrogen chloride and better on-stream efficiency of subsequent processing procedures.

One embodiment of the invention may be characterized as a process for the selective conversion and removal of organic nitrates contained in a feed stream comprising halogenated organic compounds and the undesired organic nitrates which process comprises: (a) contacting the feed stream and hydrogen with a selective hydrogenation catalyst comprising a refractory inorganic oxide and at least one metallic compound having hydrogenation activity in a hydrogenation zone at hydrogenation conditions selected to produce one or more water-soluble nitrogen compound while minimizing the production of hydrogen halide compounds; (b) separating the resulting effluent from the hydrogenation zone to produce a hydrogen-rich gaseous stream and a liquid stream comprising halogenated organic compounds and the resulting water-soluble nitrogen compounds; (c) contacting the liquid stream comprising halogenated organic compounds and water-soluble nitrogen compounds with an aqueous scrubbing solution to absorb at least a portion of the water-soluble nitrogen compounds; (d) recovering an aqueous stream comprising water-soluble nitrogen compounds; and (e) recovering a product stream comprising halogenated organic compounds having less than about 20 ppm nitrogen.

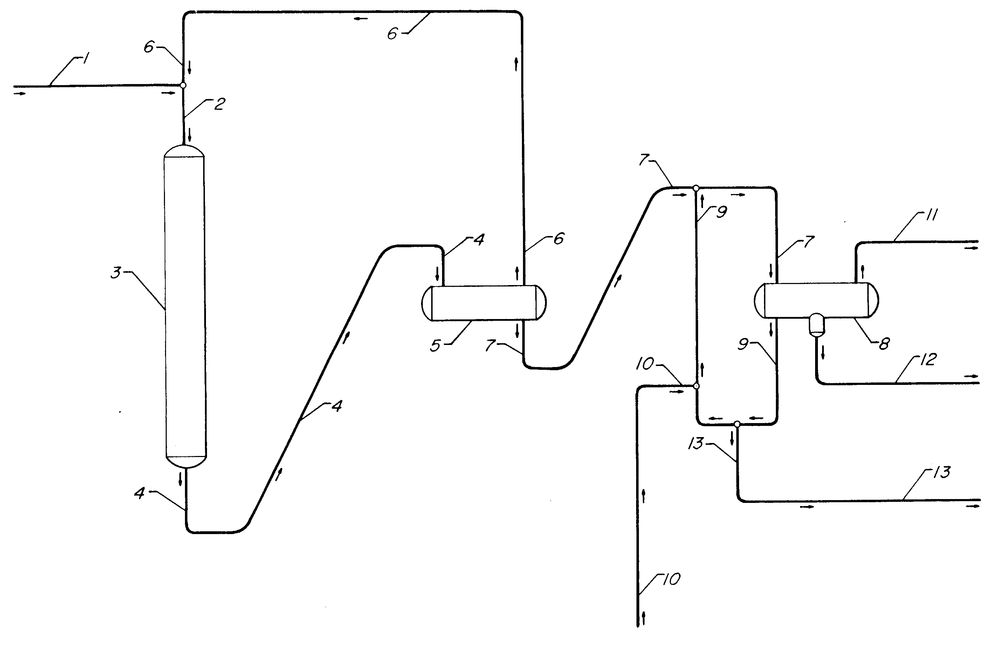

BRIEF DESCRIPTION OF THE DRAWING

The drawing is a simplified process flow diagram of a preferred embodiment of the present invention.

DETAILED DESCRIPTION

The present invention provides an improved process for the selective conversion and removal of organic nitrates from a stream containing halogenated organic compounds and organic nitrates while minimizing the production of hydrogen halide compounds. A wide variety of organic streams containing halogenated organic compounds and organic nitrates are to be candidates for feed streams in accordance with the process of the present invention. Examples of organic streams which are suitable for treatment by the process of the present invention are halogenated byproducts from propylene oxide, epichlorohydrin, acetaldehyde, vinyl chloride monomer, brominated phenol and bisphenol, synthetic refrigerants and other similar chemical production plants as well as spent halogenated solvents and residues derived from the recycle of such solvents. The organic nitrates are preferably present in the feedstock in an amount from 20 wppm to 2 weight percent. The halogenated organic compounds are preferably present in the feedstock in an amount from 1 to 99 weight percent.

In accordance with the subject invention, a feed stream comprising halogenated organic compounds and organic nitrates is contacted in the presence of hydrogen with a selective hydrogenation catalyst in a hydrogenation zone at hydrogenation conditions selected to produce at least one water-soluble nitrogen compound while minimizing the production of hydrogen halide compounds. The water-soluble nitrogen compound is preferably selected from the group consisting of ammonia, ammonium chloride, primary amines, secondary amines, tertiary amines, and nitriles. The catalytic hydrogenation zone may contain a fixed, ebullated or fluidized catalyst bed. This reaction zone is preferably maintained under an imposed pressure from atmospheric (101.3 kPa) to 2,000 psig (13891 kPa) and more preferably under a pressure from 100 psig (791 kPa) to 1000 psig (6996 kPa). Suitably, such reaction is conducted with a maximum catalyst bed temperature in the range of 60°F (15°C) to 212°F (100°C) selected to produce one or more water-soluble nitrogen compound while minimizing the production of hydrogen halide compounds. In accordance with the present invention, it is contemplated that the desired hydrogenation conversion includes primarily the selective conversion of water-insoluble organic nitrates. As used herein, the expression "organic nitrates" refers to water-insoluble compounds containing nitrogen. Preferred organic nitrates are selected from the group consisting of methyl nitrate and chloropropyl nitrate. Hydrogen is present in the hydrogenation zone in an amount at least great enough to satisfy the stoichiometric hydrogen required for the selective conversion of organic nitrates. Further preferred operating conditions include liquid hourly space velocities in the range from 0.05 hr$^{-1}$ to 20 hr$^{-1}$ and hydrogen circulation rates from 1 standard cubic feet per barrel (SCFB) (0.17 normal m$^3$/m$^3$) to 1000 SCFB (168 normal m$^3$/m$^3$), preferably from 10 SCFB (1.68 normal m$^3$/m$^3$) to 500 SCFB (84 normal m$^3$/m$^3$) when hydrogen circulation is used.

The preferred catalytic composite disposed within the hydrogenation zone can be characterized as containing a metallic component having hydrogenation activity, which component is combined with a suitable refractory inorganic oxide carrier material or carbon-based material of either synthetic or natural origin. The precise composition and method of manufacturing the carrier material is not considered essential to the present invention. Preferred carrier materials are alumina, silica and mixtures thereof. Suitable metallic components having hydrogenation activity are those selected from the group comprising the transition metals of Groups VI-B and VIII of the Periodic Table, as set forth in the Periodic Table of the Elements, E.H. Sargent and Company, 1964. Thus, the catalytic composites may comprise one or more metallic components from the group of molybdenum, tungsten, chromium, iron, cobalt, nickel, platinum, palladium, iridium, osmium, rhodium, ruthenium, and mixtures thereof. The concentration of the catalytically active metallic component, or components, is primarily dependent upon a particular metal as well as the physical and/or chemical characteristics of the particular hydrocarbon feedstock. For example, the metallic components of Group VI-B are generally present in an amount within the range of 1 to 20 weight percent, the iron-group metals in an amount within the range of 0.2 to 10 weight percent, whereas the noble metals of Group VIII are preferably present in an amount within the range of 0.1 to 5 weight percent, all of which are calculated as if these components existed within the catalytic composite in the elemental state. In accordance with a preferred embodiment of the present invention, the preferred catalysts contain alumina and palladium. It is further contemplated that hydrogenation catalytic composites may comprise one or more of the following components: cesium, francium, lithium, potassium, rubidium, sodium, copper, gold, silver, cadmium, mercury and zinc.

The resulting effluent from the selective hydrogenation zone is, in one embodiment, preferably admitted to a separation zone which is maintained at essentially the same pressure as the hydrogenation zone wherein a hydrogen-rich gaseous phase is produced and recycled to the hydrogenation zone. A liquid phase is removed from the separation zone and is contacted with an aqueous scrubbing solution and the resulting admixture is introduced into a second separation zone in order to produce a gaseous stream, a halogenated organic stream and a spent aqueous stream. The contact of the effluent from the first separation zone with the aqueous scrubbing solution may be performed in any convenient manner and is preferably conducted by co-current, in-line mixing which may be promoted by inherent turbulence, mixing orifices or any other suitable mixing means. The aqueous scrubbing solution is preferably introduced in an amount from 0.05 to 200 vol. % based on the liquid effluent from the first separation zone. The aqueous scrubbing solution is selected depending on the characteristics of the original feedstock. In accordance with the present invention, the feedstock comprises halogenated compounds and the aqueous scrubbing solution preferably contains an acid compound such as hydrogen chloride to absorb the water-soluble nitrogen compounds which are produced in the hydrogenation zone.

With reference now to the drawing, a liquid feed stream comprising halogenated organic compounds and organic nitrates is introduced into the process via conduit 1, joined by a recycle, hydrogen-rich gaseous stream which is provided via conduit 6 and the resulting admixture is introduced into hydrogenation zone 3 via conduit 2. A resulting effluent stream from hydrogenation zone 3 is removed via conduit 4 and introduced into high pressure vapor/liquid separator 5. A hydrogen-rich gaseous stream is removed from high pressure vapor/liquid separator 5 via conduit 6 and is recycled to hydrogenation zone 3. Since hydrogen is lost in the process by means of a portion of the hydrogen being dissolved in the exiting liquid stream and hydrogen being consumed during the selective conversion of the organic nitrates, it is necessary to supplement the hydrogen-rich gaseous stream with makeup hydrogen from some suitable external source, for example, a catalytic reforming unit or a hydrogen plant. Makeup hydrogen may be introduced into the system at any convenient and suitable point not shown in the drawing. A liquid stream containing halogenated organic compounds and having a reduced level of organic nitrates is removed from high pressure vapor/liquid separator 5 via conduit 7 and is contacted at the junction of lines 7 and 9 with an aqueous scrubbing solution which is supplied via conduit 9 and the resulting admixture is introduced via conduit 7 into low pressure vapor/liquid separator 8. A gaseous stream comprising dissolved hydrogen and any other light gaseous compounds present is removed from low pressure vapor/liquid separator 8 via conduit 11 and recovered. A liquid stream containing halogenated organic compounds and having a reduced level of organic nitrates is removed from low pressure vapor/liquid separator 8 via conduit 12 and recovered. An aqueous scrubbing solution is removed from low pressure vapor/liquid separator 8 via conduit 9 and is contacted with the liquid effluent from the high pressure vapor/liquid separator 5 as described hereinabove. Fresh, makeup aqueous scrubbing solution is introduced via conduit 10 into the circulating aqueous scrubbing solution which is transported via conduit 9. Spent aqueous scrubbing solution is removed from low pressure vapor/liquid separator 8 via conduit 9 and conduit 13 and is recovered.

CONTROL EXAMPLE 1

A feed stream containing chlorinated by-products from a propylene oxide production plant having the characteristics presented in Table 1 was charged at a rate of 100 mass units per hour to a hydrogenation zone containing a hydrogenation catalyst containing alumina and palladium, and operated at conditions including a pressure of 750 psig (5273 kPa), a hydrogen circulation rate of 40,000 SCFB (6720 normal $m^3/m^3$) and a catalyst peak temperature of about 572°F (300°C). The object of this example is to hydrogenate the chlorinated organic compounds to produce hydrocarbons and hydrogen chloride. After the hydrogenation zone was operated for about 35 days, the reactor circuit started to develop increasing pressure drop which indicated partial plugging and the activity of the catalyst was observed to prematurely decline. The plant was subsequently shut down and the hydrogenation zone outlet piping was inspected and found to contain significant deposits of ammonium chloride. Before the plant was shut down, a resulting liquid product was recovered from the effluent of the hydrogenation zone in an amount of about 100 mass units per hour and having the characteristics presented in Table 2.

TABLE 1

| CHLORINATED BY-PRODUCT FEEDSTOCK ANALYSIS | |
|---|---|
| Dichloropropane, weight percent | 90 |
| Epichlorohydrin, weight percent | 1 |
| Dichloropropyl Ether, weight percent | 8.9 |
| Chloropropyl Nitrate, weight percent | ~ 0.1 |
| Total Nitrogen, weight ppm | ~100 |

TABLE 2

| HYDROGENATION ZONE EFFLUENT ANALYSIS, WEIGHT PERCENT OF FEED | |
|---|---|
| Hydrogen Chloride | 63.9 |
| Propane | 38.7 |
| Other | 0.9 |
| Total | $10\overline{3.5}$ |

After the premature plant shutdown was experienced, the feed was inspected and analyzed and it was determined that the feed unexpectedly contained low quantities of organic nitrate compounds which were found to be soluble in the feed and not extractable with common extraction solvents.

EXAMPLE 1

This example was performed in accordance with the present invention. A feed stream containing chlorinated by-products from a propylene oxide production plant having the characteristics presented in Table 1 was charged at a rate of 100 mass units per hour to a hydrogenation zone containing a hydrogenation catalyst containing alumina and palladium, and operated at conditions including a pressure of 200 psig (1480 kPa), a hydrogen to feed ratio of 300 SCFB (51 normal $m^3/m^3$) and a catalyst peak temperature of about 95°F (35°C). These operating conditions were selected to convert the organic nitrate compounds to water-soluble nitrogen compounds while minimizing the production of hydrogen halide compounds. These conditions are less severe than those used in the Control Example and were selected in accordance with the present invention.

A resulting liquid product was recovered from the effluent of the hydrogenation zone in an amount of about 100 mass units per hour and was water washed to extract the water-soluble nitrogen compounds. The resulting water washed liquid was found to have the characteristics presented in Table 3.

TABLE 2

| HYDROGENATION ZONE LIQUID EFFLUENT ANALYSIS | |
|---|---|
| Dichloropropane, weight percent | 90 |
| Epichlorohydrin, weight percent | < 0.1 |
| Dichloropropyl Ether, weight percent | 9 |

TABLE 2 (continued)

| HYDROGENATION ZONE LIQUID EFFLUENT ANALYSIS | |
|---|---|
| Chloropropyl Nitrate, weight percent | < 0.01 |
| Chlorinated Propanol, weight percent | 0.9 |
| Total Nitrogen, weight ppm | < 10 |

EXAMPLE 2

The conversion process described in the Control Example was repeated with the exception that a feed having the characteristics presented in Table 3 was used. A resulting product recovered from the effluent of the hydrogenation zone was essentially the same as shown in Table 2.

The plant was continuously operated for about 60 days without any detectable increased pressure drop and the catalyst stability was observed to be superior to that in the Control Example.

**Claims**

1. A process for the selective conversion and removal of organic nitrates contained in a feed stream comprising halogenated organic compounds and said organic nitrates which process comprises:

   (a) contacting said feed stream [1] and hydrogen [6] with a selective hydrogenation catalyst comprising a refractory inorganic oxide and at least one metallic compound having hydrogenation activity in a hydrogenation zone [3] at hydrogenation conditions selected to produce one or more water-soluble nitrogen compounds while minimizing the production of hydrogen halide compounds;
   (b) separating the resulting effluent [4] from said hydrogenation zone [3] to produce a hydrogen-rich gaseous stream [6] and a liquid stream [7] comprising halogenated organic compounds and the resulting water-soluble nitrogen compounds;
   (c) contacting said liquid stream [7] comprising halogenated organic compounds and water-soluble nitrogen compounds with an aqueous scrubbing solution [9] to absorb at least a portion of said water-soluble nitrogen compounds;
   (d) recovering an aqueous stream [13] comprising water-soluble nitrogen compounds; and
   (e) recovering a product stream [12] comprising halogenated organic compounds having less than about 20 ppm nitrogen.

2. The process of Claim 1 wherein said feed stream is selected from the group consisting essentially of halogenated by-products from propylene oxide, epichlorohydrin, acetaldehyde, brominated phenol and bisphenol, synthetic refrigerant and vinyl chloride monomer production plants, spent halogenated solvents and residues derived from the recycle of such solvents.

3. The process of Claim 1 or 2 wherein said hydrogenation reaction zone is operated at conditions which include a pressure from 101 to 13891 kPa (atmospheric to 2000 psig), a hydrogen circulation rate of 0.17 to 168 normal $m^3/m^3$ (1 to 1000 SCFB) based on said stream comprising halogenated organic compounds and a maximum catalyst temperature from 15° to 100°C (60 to 212°F).

4. The process of any one of Claims 1 to 3 further characterized in that said metallic component is selected from the group comprising the transition metals of Groups VI and VIII of the Periodic Table.

5. The process of any one of Claims 1 to 4 wherein said hydrogenation catalyst comprises alumina and palladium.

6. The process any one of Claims 1 to 5 wherein said organic nitrates are present in said feed stream in an amount from 20 wppm to 2 weight percent.

7. The process of any one of Claims 1 to 6 wherein said feed stream contains halogenated organic compounds in an amount from 1 to 99 weight percent.

8. The process of any one of Claims 1 to 7 wherein said aqueous scrubbing solution is used in an amount from 0.05 to 200 volume percent based upon said liquid stream comprising halogenated organic compounds and water-soluble nitrogen compounds.

European Patent
Office

**EUROPEAN SEARCH REPORT**

Application Number

EP 97 30 0468

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| E | US 5 600 041 A (JAN CHWU-CHING ET AL) 4 February 1997 <br> * the whole document * | 1-8 | C07C17/38 |
| A | US 5 013 424 A (JAMES JR ROBERT B ET AL) 7 May 1991 <br> * claim 1 * | 1-8 | |
| D,A | US 4 895 995 A (JAMES JR ROBERT B ET AL) 23 January 1990 <br> * claim 1 * | 1-8 | |

TECHNICAL FIELDS
SEARCHED      (Int.Cl.6)

C07C

The present search report has been drawn up for all claims

| Place of search | Date of completion of the search | Examiner |
|---|---|---|
| MUNICH | 1 July 1997 | Gettins, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)